Europäisches Patentamt

European Patent Office

Office européen des brevets

(11) Publication number: **0 127 713**
**B1**

(12) **EUROPEAN PATENT SPECIFICATION**

(45) Date of publication of patent specification: **02.11.89**

(21) Application number: **83303354.1**

(22) Date of filing: **09.06.83**

(51) Int. Cl.⁴: $A\ 61\ K\ 9/50$, $A\ 61\ K\ 9/52$

(54) **Microencapsulation of living tissue and cells.**

(30) Priority: **01.06.83 CA 429460**

(43) Date of publication of application:
**12.12.84 Bulletin 84/50**

(45) Publication of the grant of the patent:
**02.11.89 Bulletin 89/44**

(84) Designated Contracting States:
**AT BE CH DE FR GB IT LI LU NL SE**

(56) References cited:
GB-A-2 094 750
GB-A-2 094 833
US-A-3 962 414
US-A-4 352 883
US-A-4 364 385

CHEMICAL ABSTRACTS, vol. 99, no. 18,
October 1983, no. 146053k, Columbus, Ohio,
US; F.V.LAMBERTI et al.: "Microencapsulation
of erythrocytes in Eudragit-RL-coated calcium
alginate"

The file contains technical information
submitted after the application was filed and
not included in this specification

(73) Proprietor: **CONNAUGHT LABORATORIES
LIMITED**
**1755 Steeles Avenue West**
**Willowdale Ontario M2N 5T8 (CA)**

(72) Inventor: **GOOSEN, Mattheus Florentius
Albertus**
**18 Laxton Avenue**
**Toronto Ontario, M6K 1K9 (CA)**
Inventor: **O'SHEA, Geraldine Margaret**
**Apt. 4, 372 Davenport Road**
**Toronto Ontario, M4B 1B4 (CA)**
Inventor: **Sun, Anthony Mein-Fang**
**4 Barkwood Crescent**
**Willowdale Ontario, M2H 3G6 (CA)**

(74) Representative: **Burford, Anthony Frederick
et al**
**W.H. Beck, Greener & Co. 7 Stone Buildings
Lincoln's Inn**
**London WC2A 3SZ (GB)**

# EP  0 127 713  B1

(56) References cited:
**IDEM**

**CHEMICAL ABSTRACTS, vol. 97, no. 14, October 1982, page 336, no. 115208u, Columbus, Ohio, US; A.M.SUN et al.: "Long-term studies of microencapsulated islets of langerhans: a bioartificial endocrine pancreas"**

## Description

The present invention is concerned with the microencapsulation of living tissue or individual cells.

Various attempts have been made to microencapsulate biologically active macromolecules, tissue and individual cells so that they remain viable and in a protected state within a semi-permeable membrane which permits passage of low molecular weight substances, such as nutrients and oxygen, but not of high molecular weight substances, such as proteins and cells. However, none of these attempts has been successful in providing microcapsules in which tissue or cells enclosed within the semi-permeable membrane are able to survive in an animal body for longer than 2 to 3 weeks, which severely limits the utility of the products in the treatment of diseases requiring organ transplantation, such as diabetes.

In "Semipermeable Microcapsules" by T. M. S. Chang, Science, *146*, 1964, 524 to 525, there is described the microencapsulation of erythrocyte hemolysate and urease in semi-permeable polyamide (nylon) membranes. These microcapsules did not survive for long when injected into the blood stream. Papers have described the preparation of semi-permeable microcapsules containing microbial cells and viable red blood cells, namely K. Mosbach and R. Mosbach, Acta Chem. Scand., *20*, 1966, 2807 to 2812 and T. M. S. Chang, F. C. MacIntosh and S. G. Mason, "Semi-permeable Aqueous Microcapsules", Can. J. Physiol. and Pharmacology, *44*, 1966, 115 to 128. The Chang et al article mentions for the first time the possibility of using injections of encapsulated cells for organ replacement therapy.

The next significant development was the use of calcium and aluminum alginate gels for the immobilization of microbial cells and enzymes. The cells were immobilized under extremely mild conditions, thus maintaining their viability. This work was described in V. Hackel, J. Klein, R. Megret and F. Wagner, Europ. J. Appl. Microbiol *1*, 1975, 291 to 296 and M. Kierstan and C. Bucke, "The Immobilization of Microbial Cells, Subcellular Organelles, and Enzymes in Calcium Alginate Gels", Biotechnology and Bioengineering, *19*, 1977, 387 to 397.

Subsequently, viable tissue and cells were immobilized in alginate droplets coated with polylysine (F. Lim and R. D. Moss, "Microencapsulation of Living Cells and Tissues", J. Pharm. Sci. *70*, 1981, 351 to 354). While the cells remained viable in culture for up to two months, no experiments are described to test the in-vivo biocompatibility of the polylysine membrane. At approximately the same time, there was reported for the first time, the use of microencapsulated islets to correct the diabetic state of diabetic animals, in F. Lim and A. M. Sun, "Microencapsulated Islets as Bioartificial Pancreas", Science, *210*, 1980, 908 to 909. However, the microcapsules, consisting of an inner alginate core, followed by a polylysine coat and an outer polyethyleneimine membrane, were rejected by an animal body within 2 to 3 weeks of implantation due to the poor biocompatibility of the outer polyethyleneimine membrane.

Formation of the latter microcapsules also is described in US—A—4,352,883 F. Lim. As set forth therein, finely divided living tissue is suspended in an aqueous medium which contains sodium alginate, the suspension is formed into droplets of a size to envelop tissue, the droplets are gelled by conversion to calcium alginate to form discrete, shape-retaining temporary capsules, a permanent semi-permeable membrane of, for example, polyethyleneimine is formed about the temporary capsules, and the calcium alginate gel is reliquified within the membrane by ion exchange. Example 3 of the patent describes injection of the microcapsules into diabetic rats. Polyethyleneimine contains imino groups, which induce granuloma, resulting in an inflammatory response from the body, which, in turn, destroys the polymer. Polyethyleneimine, therefore, is not biocompatible and the microcapsules are ineffective for organ replacement therapy for a period lasting longer than 2 to 3 weeks.

US—A—4,352,883 mentions the possibility of using polylysine, a much more biocompatible material, instead of polyethyleneimine as the membrane. Polylysine is positively charged and it is well known that positively-charged surfaces are excellent substrates for cell growth. Cell growth on the surface of the microcapsules, such as would occur with a polylysine membrane, would transform the semi-permeable capsular wall to an impermeable one, resulting in the death of the encapsulated tissue.

It is known from US—A—4,352,883, GB—A—2,094,833 and GB—A—2,094,750 that the membrane of a biocompatible microcapsule containing living tissue or cell can be formed by the interaction of a "crosslinking" polymer with a gelled gum vehicle of a temporary capsule containing the tissue or cell. The exemplified polymers include polylysine and, preferably, polyethyleneimine. Optionally, any tendency of the microcapsules to clump can be overcome by treating the capsules with dilute aqueous sodium alginate to "tie up" free amino groups on the membrane.

Polyethyleneimine clearly is preferred over polylysine because, unlike polylysine, it is not readily digested in vivo. In particular, US—A—4,352,883 specifically teaches that the viable life of polylysine encapsulated tissue can be extended by further coating with polyethyleneimine. The use of a first coat of polylysine and an overcoat of polyethyleneimine also is referred to in GB—A—2,094,833 and GB—A—2,094,750.

None of US—A—4,352,883, GB—A—2,094,873 and GB—A—2,094,750 specify the thickness of membrane used. However, it is apparent from the exemplified methods in these references that the coating is substantially below 54 micrometres. Thus, in US—A—4,352,883, a coating formed by two minutes reaction with a 0.0167% polylysine solution (in physiological saline) is stated to provide an appropriate membrane (see column 7, lines 49/54). In GB—A—2,094,833, Example 2 forms a membrane by three minutes reaction with 0.005% aqueous polylysine. Example 5 uses 0.016% aqueous polylysine but does not specify the reaction time. In the absence of instruction to the contrary, it is reasonable to assume that said reaction time is consistent with that usually employed by Franklin Lim (i.e., 2 or 3 minutes). In GB—A—2,094,750, 0.005% aqueous polylysine is used for three minutes. Lim and Sun reported in Science *210* (1980), 908—909 the use of 0.02% polylysine for three to five minutes.

It is apparent therefore, that there is a need for the development of microcapsules which can be implanted into an animal body and be effective in the treatment of diseases requiring organ transplantation, such as, diabetes, for extended periods of time.

In accordance with the present invention, there is provided a biocompatible microcapsule suitable for implantation into an animal body and having a diameter of 50 to 2000 micrometers, said microcapsule comprising a spherical macromolecular core containing living tissue or individual cells thereof, said core being surrounded by a biocompatible semi-permeable membrane consisting of interpenetrating layers of ionically interacted biocompatible polyamino acid and a reversible gellable water-soluble polymeric substance having free acid groups and being permeable to and permitting nutrients and oxygen to flow from a body in which the microcapsule is implanted to the said living tissue or individual cells thereof and permitting metabolic products of said tissue or cells to flow therefrom to said body and being impermeable to said tissue or cells, characterised in that the membrane has a thickness of 5 to 20 micrometers, is in the form of a hydrogel having an overall water content within the membrane structure of at least 20 weight percent, has an outer biocompatible negatively-charged surface formed by ionic reaction of free amino groups of said polyamino acid with a polymeric material having free negatively charged groups, and resists degradation and remains permeable in vivo for at least two months.

The invention also provides a method of encapsulating a core material with a semi-permeable membrane to form microcapsules having a diameter of 50 to 2000 micrometers, said method comprising the steps of (a) placing the material in an aqueous solution of water-soluble polymeric substance that can be reversibly gelled and which has free acid groups; (b) forming the solution into droplets; (c) gelling the droplets to produce discrete shape-retaining temporary capsules; (d) forming semi-permeable membranes about the temporary capsules by contact between the temporary capsules and a cationic polymer containing free amino groups to cause ionic reaction with the acid groups in a surface layer of the capsule, and (e) contacting the microcapsules formed in step (d) with a biocompatible anionic polymer material which contains free negatively-charged groups capable of ionic reaction with the free amino groups in a surface layer of the microcapsule, characterised in that said anionic polymer forms on the microcapsules an outer coating having a negatively-charged outer surface and in that the membrane is a hydrogel having an overall water content within the membrane structure of at least 20 weight percent and the contact with the cationic polymer is sufficient to form a membrane which has a thickness of 5 to 20 micrometers and resists degradation and remains permeable in vivo for at least two months.

In the present invention, the core material is encapsulated in a biocompatible semi-permeable membrane in the form of a hydrogel. The material to be encapsulated is suspended in a physiologically-compatible medium containing a water soluble substance which can be reversibly gelled to provide a temporary protective environment for the tissue. The medium is formed into droplets containing the tissue and gelled, for example, by changing conditions of temperature, pH or ionic environment, to form temporary capsules of substantially perfect spherical shape so as to provide an overall improved physical strength when compared with microcapsules formed from non-spherical capsules. Thereafter, the temporary capsules which result are treated to form a membrane of controlled permeability about the shape-retaining temporary capsules. The semi-permeable nature of the membrane permits nutrients and oxygen to flow to the core material and metabolic products to flow therefrom while retaining the core material within the microcapsule. The biocompatible nature of the semi-permeable membrane allows the passage of such materials to and from the core to occur without inflammation or other adverse body response while the outer negatively-charged surface inhibits surficial cell growth, so that the membrane remains semi-permeable and effective for extended periods of time, typically from three to six months or longer.

The temporary capsules may be formed from any non-toxic water-soluble substance that can be gelled to form a shape retaining mass by a change of conditions in the medium in which it is placed, and also comprises plural groups that are readily ionized to form anionic or cationic groups. The presence of such groups enables surface layers of the capsule to cross-link to produce a permanent membrane when exposed to polymers containing multiple functionalities of the opposite charge.

Preferably the temporary capsules are formed from a polysaccharide gum, which may be natural or synthetic, of a type that can be gelled to form a shape retaining mass by exposure to a change in conditions and can be permanently cross-linked or hardened by polymers containing reactive groups, such as amino groups, which can react with the acidic polysaccharide constituents. Most preferably, the gum is alkali metal alginate, specifically sodium alginate, although other water-soluble gums may be used.

The temporary capsules may be formed from sodium alginate by extruding droplets of aqueous sodium alginate solution into an aqueous calcium chloride solution. It has been found that substantially perfectly spherical temporary capsules can be formed by using an aqueous sodium alginate solution having a viscosity of at least 30 mPa.s. At viscosities below this critical lower limit, the temporary capsules have an irregular shape. Perfectly spherical capsules are obtained over a wide range of viscosity of the sodium alginate solution, with an upper limit being dictated largely by the ability to extrude the solution into the hardening medium. Usually, the viscosity of the aqueous sodium alginate solution does not exceed 1000 mPa.s.

Formation of the permanent semi-permeable membrane about the temporary capsules is effected by ionic reaction between free acid groups in the surface layer of the gelled gum and amino groups of a biocompatible polyamino acid, typically in a dilute aqueous solution of the selected polymer.

Cross-linking biocompatible polymers used are polylysine and other polyamino acids. It is noted that polyethyleneimine and other imine-containing polymers are unsuitable for membrane formation in view of their non-biocompatible nature. The molecular weight of the polyamino polymer may vary widely, depending on the degree of permeability required, and typically is in the range of 11,000 to 400,000, preferably 11,000 to 100,000. The use of polylysine or other polyamino acid results in microcapsules having a positively-charged surface, which, as already noted, would be unsuitable for long term viability, although the microcapsules are biocompatible.

In accordance with the present invention, the semi-permeable membrane is treated with a non-toxic biocompatible water-soluble polymeric material which is capable of ionic reaction with free amino groups to form an outer negatively-charged coating about the membrane, typically by suspension of the microcapsules in an aqueous solution of the polymeric material. The material used to form the outer coating preferably is the same material as is used to form the temporary capsules, preferably a polysaccharide gum, more preferably an alkali metal alginate, such as sodium alginate. Other biocompatible polymeric materials containing base-reactive groups, such as polyvinyl alcohol, polylactic acid, poly glycolic-lactic acid copolymers and poly beta-hydroxy butyric acid, may be used to form the outer coating to the microcapsules. Molecular weights of such polymeric materials typically vary from $10^4$ to $10^6$.

The treatment of the polyamino microcapsules with the biocompatible base-reactive material retains the overall biocompatible nature of the semi-permeable membrane and, more importantly, results in a negatively-charged outer surface which inhibits cell growth and, therefore, permits the semi-permeable membrane to retain its permeability and hence effectiveness over an extended period of time.

Following formation of the microcapsules, reliquification of the suspending medium for the core material may be effected by re-establishing the conditions under which the material is liquid. This may be achieved by ion exchange to remove multivalent cation, for example, by immersion in phosphate buffered saline or citrate buffer.

The process of the invention may be used to encapsulate living tissue, multicellular fractions thereof or individual cells, for example, islets of Langerhans, liver cells and red blood cells. The microcapsules which result may be implanted into an appropriate site within a mammalian body for the purpose of providing the body with the specialized physiological function of the tissue while the tissue remains viable. The implantation may be achieved by simple injection, so that surgical procedures are not required.

The biocompatible semi-permeable membrane encapsulating the core material consists of interpenetrating layers of ionically-interacted biocompatible materials. The overall wall thickness of the semi-permeable membrane varies from 5 to 20 μm. The microcapsules themselves usually have a diameter in the range of 50 to 2000 μm, preferably in the range of 200 to 1000 μm for microcapsules containing islets of Langerhans as the core material. The biocompatible semi-permeable membrane is in the form of a hydrogel and hence has an overall water content within the membrane structure of at least 20 weight percent, which may vary up to 90 weight percent in the surface region.

The materials which are used to form the biocompatible semi-permeable membrane are biodegradable by the body into which the microcapsules are implanted. Such biodegradation takes place over the active life of the microcapsules and is responsible for the ultimate failure of the microcapsules. The biodegradation is a very slow process, as is evidenced by observed effectiveness of the control of blood sugar in rats by microencapsulated islets of Langerhans of at least three months and, in some cases, as long as one year.

In a particularly preferred embodiment of the invention, living cells are microencapsulated within a polylysine-alginate semi-permeable hydrogel by suspending cells uniformly in a sodium alginate solution in physiological saline. Where the microcapsules are to be used for the treatment of diabetes by controlling blood sugar in animals, including humans, the living cells take the form of islets of Langerhans from an animal pancreas.

Spherical droplets containing the cells are produced from an aqueous sodium alginate solution by a droplet generator, such as, syringe pump extrusion or electrostatic extrusion, and are collected as gelled spheres in a hardening solution, such as, calcium chloride. The microcapsules then are coated with polylysine followed by an outer coating of sodium alginate. The microcapsules may then be suspended in isotonic sodium citrate or other convenient ion exchange medium to reliquify the alginate gel inside the microcapsule.

The outer biochemically inert but biocompatible alginate surface is a negatively-charged hydrogel containing up to 90 percent water. The low interfacial tension between the swollen gel surface and the aqueous biological environment minimizes protein interaction, otherwise a strong protein-polymer interaction may cause a severe inflammatory response. The biocompatibility of the hydrogel membrane leads to long term viability of the capsules when implanted. Polyethyleneimine-surfaced microcapsules do not appear to possess this property and hence are rejected by the body and produce a strong inflammatory

5

response, which severely limits the useful life of the microcapsules within the body. The soft rubbery consistency of most hydrogels may also contribute to their biocompatibility by decreasing frictional irritation to surrounding tissues.

The durability of the microcapsules can be increased further by increasing the thickness of the polylysine membrane, as compared with the thickness of the polylysine-polyethyleneimine membrane used in US—A—4,352,883. The strength of the microcapsules also may be increased by cross-linking, for example, using glutaraldehyde, prior to reliquification of the gel.

In the present invention, it is not essential that the biocompatible outer surface be composed of sodium alginate, but it is essential that the outer surface be biocompatible and negatively-charged. Binding occurs between the negatively-charged groups, usually hydroxyl or carboxyl groups, and the positively-charged amino groups on polylysine.

The permeability of the microcapsule to nutrients and metabolic products may be varied by varying the molecular weight of the polylysine used in forming the semi-permeable membrane. Usually, the molecular weight of the polylysine varies from 11,000 to 400,000, preferably 11,000 to 100,000. Higher molecular weights lead to greater permeability than lower molecular weights.

The invention is illustrated by the following Examples:

## Example 1

This Example illustrates the microencapsulation of islets of Langerhans.

Cultured rat islets of Langerhans ($2 \times 10^3$ islets in 0.2 ml medium) were suspended uniformly in 2 ml of 1.5% (w/w) sodium alginate solution (viscosity 51 mPa.s) in physiological saline. Spherical droplets containing islets were produced by syringe pump extrusion through a 0.56 mm (22-gauge) needle and collected in 1.5% (w/w) calcium chloride solution. The supernatant was decanted and the gelled spherical alginate droplets, containing islets, were washed with dilute CHES (2-cyclohexylamino-ethane sulfonic acid) solution and 1.1% calcium chloride solution.

After aspirating off the supernatant, the gelled droplets were incubated for exactly 6 minutes in 0.05% (w/w) polylysine having a molecular weight of 25,000. (These conditions are a significant increase in incubation time and polylysine concentration compared to previous procedures, wherein, in US—A—4,352,883, Lim used 0.013% polylysine and 3 minutes incubation time, and, in their reported work, Lim and Sun used 0.02% polylysine and 3 to 5 minutes incubation time. These changes result in a stronger polylysine membrane).

The supernatant was decanted and the polylysine capsules were washed with dilute CHES, 1.1% calcium chloride solution and physiological saline. The washed polylysine capsules were incubated for 4 minutes in 30 ml of 0.03% sodium alginate to permit the formation of an outer alginate membrane on the initial polylysine membrane, by ionic interaction between the negatively charged alginate and the positively charged polylysine.

The resulting microcapsules were washed with saline, 0.05M citrate buffer for 6 minutes to reliquify the inner calcium alginate, and a final saline wash. The microcapsules were found to be perfectly spherical and each to contain from 1 to 2 viable islets. The microcapsules had diameters varying from 200 to 1000 µm and wall thicknesses varying from 5 to 10 µm. The microcapsules were suspended in nutrient medium at 37°C.

The experiment was repeated with islet cells from mouse, bovine and dog pancreas and similar microencapsulated products were formed.

## Example 2

This Example illustrates the viability of the microencapsulated islets.

In perifusion experiments, the insulin secretion from the microencapsulated rat islets produced in accordance with the procedure of Example 1 was determined to be comparable with that from unencapsulated islets. When the glucose concentration was raised from 50 to 300 mg, there was a biphasic response of insulin release from both groups of islets and the insulin secretion increased.

The increase in the quantity of insulin in the presence of a high glucose concentration clearly demonstrated that the viability and functionality of the cells were retained throughout the process of microencapsulation.

After 2 months in culture at 37°C, the microencapsulated islets were observed to have remained morphologically and functionally intact.

## Example 3

This Example illustrates the injection of microencapsulated islets into diabetic rats.

Diabetic rats with blood glucose levels in the range of 370 to 470 mg/dl were treated with approximately $3 \times 10^3$ rat islets microencapsulated as set forth in Example 1. The microcapsules were introduced by injection into the peritoneal cavity using a 0.41 mm (16-gauge) needle fitted into a syringe.

Unencapsulated islets and islets microencapsulated in a polylysine-polyethyleneimine membrane, produced as described in US—A—4,352,883 (Lim), were used as controls. Blood glucose levels were assayed twice per week to determine the period of time for which the blood glucose level was lowered. The results obtained are set forth in the following Table I:

TABLE I

| Membrane Type | Number of Weeks Blood Glucose Level Lowered | |
|---|---|---|
| None | 1 | (N = 4) |
| Polylysine polyethyleneimine (Lim Patent) | 2 to 3 | (N = 8) |
| Polylysine alginate (Present invention) | 13 to 52 | (N = 10) |

As can be seen from the results of Table I, the islets enclosed in the biocompatible polylysine alginate membranes of the invention survived up to 52 weeks, as demonstrated by the normal blood sugar levels in the diabetic rats. In contrast, the islets enclosed in the polylysine-polyethyleneimine capsular membranes of the Lim Patent showed survival times of less than 3 weeks.

Example 4

This Example shows the effect of multiple injections of microencapsulated islets.

The procedure of Example 3 was repeated except that, following a return to hyperglycemia (blood sugar concentration greater than 300 mg/ml), a second injection of polylysine alginate microencapsulated islets produced in accordance with the procedure of Example 1 normalized the blood sugar level of the animal for a longer period than the initial injections, allowing the blood sugar level of the diabetic rats to be controlled for longer than six months with just two injections.

In contrast, five injections of polylysine-polyethyleneimine microencapsulated islets at 2 to 3 week intervals were barely able to control the blood glucose level of diabetic animals for three months (N = 8).

Example 5

This Example illustrates the injection of microencapsulated rat islets into diabetic mice.

The procedure of Example 3 was repeated except that fewer islets were used (1000 rat islets) and diabetic mice were employed. No polylysine polyethyleneimine microcapsules were used as controls.

Blood sugar levels in the diabetic mice were controlled for more than two months with a single injection (I.P.), indicating that xenograft transplants (cross-species) are possible.

Example 6

This Example illustrates the viability of recovered microencapsulated transplanted islets.

Microencapsulated islets were recovered from some of the treated diabetic rats in Example 3 at 3, 5 and 12 months postimplantation. The majority of the microcapsules were still physically intact and contained viable insulin-secreting islets, as demonstrated by secretion of insulin from the recovered islets in culture in response to a high glucose concentration.

Example 7

This Example illustrates the microencapsulation of liver cells.

The procedure of Example 1 was repeated, except that liver cells were employed in place of islets. An electrostatic droplet generator was employed in place of the syringe pump extruder to produce smaller capsules of diameter from 100 to 300 μm. Capsules containing viable liver cells were obtained, as determined by trypan blue exclusion and a histological study. Each capsule was observed to contain about 300 liver cells.

Example 8

This Example illustrates the use of polyvinyl alcohol as the external surface of the microcapsules.

The procedure of Example 1 was repeated, except that 1.0% (w/w) solution of polyvinyl alcohol in phosphate buffered saline was used in place of the sodium alginate solution for formation of the outer membrane coating. The polyvinyl alcohol did not significantly alter the permeability of the capsular membrane.

Polyvinyl alcohol is known to be a biocompatible water-soluble polymer and has been used in many surgical applications, such as, thromboresistant coatings for artificial blood vessels, and hence the microcapsules produced in this Example are expected to exhibit similar blood sugar decreasing capability in diabetic animals to the microcapsules produced by the procedure of Example 1.

Example 9

This Example illustrates the use of polylactic acid as the external surface of the microcapsules.

The procedure of Example 1 was repeated, except that 0.1% (w/w) solution of polylactic acid in buffered saline was used in place of the sodium alginate solution for formation of the outer membrane coating. The polylactic acid was initially dissolved in dilute sodium hydroxide and then neutralized with hydrochloric acid. The ongoing viability of the islets in the microcapsules so produced was demonstrated with trypan blue staining. Polylactic acid is a biocompatible polymer that is currently in clinical use as suture material. It is expected, therefore, that the microcapsules produced in this Example will exhibit similar blood sugar decreasing capability in diabetic animals to the microcapsules produced by the procedure of Example 1.

Example 10

This Example illustrates the preparation of spherical calcium alginate droplets.

Sodium alginate solutions of varying concentrations (and hence viscosities) were extruded with a syringe pump through a 0.56 mm (22 gauge) needle into a 1.5% (w/w) calcium chloride hardening solution and the resulting gel droplets were collected and their physical shape observed. The results are reproduced in the following Table II:

TABLE II

| Sodium Alginate % (w/w) | Viscosity (mPa.s) | Fractions of Droplets which are Spherical (%) |
|---|---|---|
| 1.5 | 51 | 100 |
| 1.4 | 43 | 100 |
| 1.3 | 36 | 100 |
| 1.2 | 30 | 100 |
| 1.1 | 25 | 25 |
| 1.0 | 20 | 0 |
| 0.9 | 16 | 0 |
| 0.7 | 11 | 0 |
| 0.3 | 4 | 0 |

While in all instances, the droplets could be broadly described as "spheroidal", it will be apparent from Table I that it is only at concentrations of sodium alginate solution of 1.2% w/w and above, i.e., viscosities of 30 mPa.s and above, that perfect spheres are formed.

Example 11

This Example illustrates variation of the microcapsule permeability.

The procedures of Examples 1, 8 and 9 were repeated, except that the molecular weight of the polylysine was varied, with microcapsules being produced from polylysine of molecular weight from 11,000 up to 400,000. The permeability of the resulting microcapsules was determined by the diffusion of serum albumin or $^{125}$I.Ig G (antibody) into and out of the microcapsules.

It was found that the use of the 400,000 molecular weight polylysine increased the permeability of the microcapsules while the use of the 11,000 molecular weight polylysine decreased the permeability of the microcapsules.

Capsules prepared using 0.075 wt% of mixed molecular weight polylysine in the process of Example 1, comprising 10 mg polylysine of 25,000 molecular weight of 5 mg of polylysine of 4,000 molecular weight were found to be less permeable to lysed red blood cells, when compared to capsules prepared with 0.075 wt% of polylysine of 25,000 molecular weight.

It was further found that the microcapsules having a polylactic acid outer coating had a greater permeability than the alginate and polyvinyl alcohol coated microcapsules at the same polylysine molecular weight.

The procedure of Example 1 was again repeated, except that the concentration of polylysine was doubled to 0.1% w/w and the contact time was doubled to 12 minutes, thereby increasing the thickness of the polylysine layer from about 5 μm to about 20 μm. The resulting microcapsules exhibit decreased permeability when compared to those produced in Example 1.

Example 12

This Example illustrates increasing the strength of the microcapsules.

The procedure of Examples 1, 8 and 9 were repeated, except that the microcapsules were placed in contact with 0.01% w/w glutaraldehyde for less than 60 seconds, just after the polylysine coating step or

EP 0 127 713 B1

just before the citrate washing step. The microcapsules which result are more difficult to break physically (using fine tweezers) and also are more difficult to dissociate in a heparin solution, when compared with uncross-linked material.

**Claims**

1. A biocompatible microcapsule suitable for implantation into an animal body and having a diameter of 50 to 2000 micrometers, said microcapsule comprising a spherical macromolecular core containing living tissue or individual cells thereof, said core being surrounded by a biocompatible semi-permeable membrane consisting of interpenetrating layers of ionically interacted biocompatible polyamino acid and a reversibly gellable water-soluble polymeric substance having free acid groups and being permeable to and permitting nutrients and oxygen to flow from a body in which the microcapsule is implanted to the said living tissue or individual cells thereof and permitting metabolic products of said tissue or cells to flow therefrom to said body and being impermeable to said tissue or cells, characterised in that the membrane has a thickness of 5 to 20 micrometers, is in the form of a hydrogel having an overall water content within the membrane structure of at least 20 weight percent, has an outer biocompatible negatively-charged surface formed by ionic reaction of free amino groups of said polyamino acid with a polymeric material having free negatively charged groups, and resists degradation and remains permeable in vivo for at least two months.

2. A microcapsule as claimed in Claim 1, wherein the core material is selected from islets of Langerhans, liver tissue or individual cells thereof.

3. A microcapsule as claimed in Claim 2, wherein the living tissue is islets of Langerhans or a fraction thereof whereby insulin flows from the microcapsule, and said semi-permeable membrane remains effective for at least three months on implantation of the microcapsule to control blood sugar levels in the body in which the microcapsule is implanted.

4. A microcapsule as claimed in any one of the preceding claims, wherein said reversibly gellable water-soluble polymeric material is a polysaccharide.

5. A microcapsule as claimed in Claim 4, wherein said polysaccharide is an alginate.

6. A microcapsule as claimed in any one of the preceding claims, wherein said polyamino acid is polylysine.

7. A microcapsule as claimed in any one of said claims wherein said negatively charged polymeric material is a polysaccharide, polyvinyl alcohol or polylactic acid.

8. A microcapsule as claimed in Claim 7, wherein the said polysaccharide is an alginate.

9. A method of encapsulating a core material with a semi-permeable membrane to form microcapsules having a diameter of 50 to 2000 micrometers said method comprising the steps of (a) placing the material in an aqueous solution of water-soluble polymeric substance that can be reversibly gelled and which has free acid groups; (b) forming the solution into droplets; (c) gelling the droplets to produce discrete shape-retaining temporary capsules; (d) forming semi-permeable membranes about the temporary capsules by contact between the temporary capsules and a cationic polymer containing free amino groups to cause ionic reaction with the acid groups in a surface layer of the capsule, and (e) contacting the microcapsules formed in step (d) with a biocompatible anionic polymer material which contains free negatively-charged groups capable of ionic reaction with the free amino groups in a surface layer of the microcapsule, characterised in that said anionic polymer forms on the microcapsules an outer coating having a negatively-charged outer surface and in that the membrane is a hydrogel having an overall water content within the membrane structure of at least 20 weight percent and the contact with the cationic polymer is sufficient to form a membrane which has a thickness of 5 to 20 micrometers and resists degradation and remains permeable in vivo for at least two months.

10. A method as claimed in Claim 9, wherein the core material comprises living tissue or individual cells thereof in finely divided suspended form in said aqueous solution in step (a).

11. A method as claimed in Claim 10, wherein said living tissue comprises islets of Langerhans, whereby the microcapsules produced by the method can be used to control blood sugar levels in diabetic animal bodies into which they are implanted.

12. A method as claimed in any one of Claims 9 to 11, wherein said reversibly gellable water-soluble polymeric substance is a polysaccharide gum.

13. A method as claimed in Claim 12, wherein said gum is an alkali metal alginate.

14. A method as claimed in any one of Claims 9 to 13, wherein said cationic polymer has a molecular weight of 11,000 to 400,000 daltons.

15. A method as claimed in Claim 14, wherein said cationic polymer has a molecular weight of 11,000 to 100,000 daltons.

16. A method as claimed in any one of Claims 9 to 15, wherein said cationic polymer is polylysine.

17. A method as claimed in any one of Claims 9 to 16, wherein said biocompatible anionic polymeric material comprises a polysaccharide gum containing free acid groups.

9

18. A method as claimed in Claim 17, wherein said polysaccharide gum is sodium alginate.

19. A method as claimed in any one of Claims 9 to 16, wherein said biocompatible anionic polymeric material is a polyvinyl alcohol having free hydroxyl groups or a polylactic acid containing free acid groups.

20. A method as claimed in any one of Claims 9 to 18, wherein said reversibly gellable water-soluble substance comprises sodium alginate, said cationic polymer comprising polylysine and said anionic polymer comprises sodium alginate.

21. A method as claimed in any one of Claims 9 to 18 and 20, wherein said reversibly gellable water-soluble substance is sodium alginate and the viscosity of the aqueous solution of sodium alginate used in step (a) is at least sufficient to result in the formation of substantially spherical temporary capsules.

22. A method as claimed in Claim 21, wherein said aqueous sodium alginate solution has a viscosity of at least 30 mPa.s.

23. A method as claimed in any one of Claims 9 to 22, including the further step of reliquifying the gel within the semi-permeable membrane following step (e).

**Patentansprüche**

1. Biologisch verträgliche Mikrokapsel, geeignet zum Implantieren in einen Tierkörper, mit einem Durchmesser von 50 bis 2000 Micrometer (µm), wobei die Mikrokapsel einen sphärischen makromolekularen Kern aufweist, der lebendes Gewebe oder einzelne Zellen enthält, und der Kern von einer biologisch verträglichen semipermeablen Membran umgeben ist, die aus durchdrungenen Schichten aus einer ionisch zusammenwirkenden, biologisch verträglichen Polyaminosäure, und einer reversibel gelierenden wasserlöslichen polymeren Substanz mit freien Säuregruppen besteht, wobei die polymere Substanz durchlässig für Nährstoffe und Sauerstoff ist, die von dem Körper, in den die Mikrokapsel implantiert ist, in das lebende Gewebe oder die einzelnen Zellen einfließen und durchlässig für metabolische Produkte des Gewebes oder der Zellen ist, die wieder zurück in den Körper fließen und undurchlässig für das Gewebe oder die Zellen ist, dadurch gekennzeichnet, daß die Membran eine Dicke von 5 bis 20 Micrometer (µm) aufweist, die Form eines Hydrogels aufweist, mit einem Gesamtwasservolumen innerhalb der Membranstruktur von mindestens 20 Gew.-%, daß die Membran eine äußere biologisch verträgliche negativ geladene Oberfläche aufweist, die gebildet wird durch eine ionische Reaktion von freien Aminogruppen der Polyaminosäure, mit einer polymeren Substanz, die freie negativ geladene Gruppen aufweist und einem Abbau widersteht und im lebenden Körper für wenigstens zwei Monate durchlässig bleibt.

2. Mikrokapsel nach Anspruch 1, dadurch gekennzeichnet, daß das Kernmaterial aus Langerhans'schen Inseln, Lebergewebe oder individuellen Zellen ausgewählt ist.

3. Mikrokapsel nach Anspruch 2, dadurch gekennzeichnet, daß das lebende Gewebe aus Langerhans'schen Inseln oder Fraktionen davon besteht, wodurch Insulin aus der Mikrokapsel ausfließt und die semipermeable Membran über wenigstens drei Monate nach der Implantation der Mikrokapsel wirksam ist, und den Blutzuckerspiegel in dem die Mikrokapsel beinhaltenden Körper steuert.

4. Mikrokapsel nach einem der vorhergehenden Ansprüche, dadurch gekennzeichnet, daß die reversibel gelierende wasserlösliche polymere Substanz ein Polysaccharid ist.

5. Mikrokapsel nach Anspruch 4, dadurch gekennzeichnet, daß das Polysaccharid ein Alginat ist.

6. Mikrokapsel nach einem der vorhergehenden Ansprüche, dadurch gekennzeichnet, daß die Polyaminosäure ein Polylysin ist.

7. Mikrokapsel nach einem der vorhergehenden Ansprüche, dadurch gekennzeichnet, daß die negativ geladene polymere Substanz ein Polysaccharid, Polyvinylalkohol oder eine Polymilchsäure ist.

8. Mikrokapsel nach Anspruch 7, dadurch gekennzeichnet, daß das Polysaccharid ein Alginat ist.

9. Verfahren zum Einkapseln von Kernmaterial in eine semipermeable Membran zur Herstellung von Mikrokapseln mit einem Durchmesser von 50 bis 2000 Micrometer (µm), wobei das Verfahren folgende Schritte umfaßt:

(a) Einbringen des Materials in eine wässrige Lösung einer wasserlöslichen polymeren Substanz, die reversibel gelierbar ist und die freie Säuregruppen aufweist;

(b) Formen der Lösung zu Tröpfchen;

(c) Gelieren der Tröpfchen zum Herstellen von diskreten formbeständigen, vorläufigen Kapseln;

(d) Formen von semipermeablen Membranen un die vorläufigen Kapseln durch Kontaktieren der vorläufigen Kapseln mit einem kationischen, freie Aminogruppen enthaltenden Polymer, um in einer Oberflächenschicht der Kapsel eine ionische Reaktion mit den Säuregruppen zu verursachen; und

(e) Kontaktieren der gemäß dem Verfahrensschritt (d) hergestellten Mikrokapseln mit einer biologisch verträglichen anionischen polymeren Substanz, welche freie negativ geladene Gruppen enthält, die die Fähigkeit einer ionischen Reaktion mit den freien Aminogruppen in einer Oberflächenschicht der Mikrokapsel besitzen, dadurch gekennzeichnet, daß

das anionische Polymer auf den Mikrokapseln einen äußeren Überzug bildet, welcher eine negativ geladene Außenoberfläche aufweist, und daß die Membran ein Hydrogel ist, das einen Gesamtwasseranteil innerhalb der Membranstruktur von mindestens 20 Gew.-% aufweist, und daß der Kontakt mit dem kationischen Polymer ausreicht, eine Membran zu bilden, die eine Dicke von 5 bis 20 Micrometer (µm) besitzt und einem Abbau widersteht und im lebenden Körper wenigstens zwei Monate durchlässig bleibt.

10. Verfahren nach Anspruch 9, dadurch gekennzeichnet, daß das Kernmaterial lebendes Gewebe oder einzelne Zellen enthält, die in der wässrigen Lösung des Verfahrensschritts (a) fein verteilt suspendiert sind.

11. Verfahren nach Anspruch 10, dadurch gekennzeichnet, daß das lebende Gewebe Langerhans'sche Inseln enthält, wodurch die gemäß diesem Verfahren hergestellte Mikrokapseln dafür verwendet werden können, den Blutzuckerspiegel innerhalb des Körpers eines zuckerkranken Tieres, in den sie implantiert worden sind, zu steuern.

12. Verfahren nach einem der Ansprüche 9 bis 11, dadurch gekennzeichnet, daß die reversibel gelierende wasserlösliche polymere Substanz ein polysaccharider Gummi oder Harz ist.

13. Verfahren nach Anspruch 12, dadurch gekennzeichnet, daß der Gummi oder das Harz ein Alkalimetallalginat ist.

14. Verfahren nach einem der Ansprüche 9 bis 13, dadurch gekennzeichnet, daß das kationische Polymer ein Molekulargewicht von 11000 bis 40000 Dalton aufweist.

15. Verfahren nach Anspruch 14, dadurch gekennzeichnet, daß das kationische Polymer ein Molekulargewicht von 11000 bis 100000 Dalton aufweist.

16. Verfahren nach einem der Ansprüche 9 bis 15, dadurch gekennzeichnet, daß das kationische Polymer ein Polylysin ist.

17. Verfahren nach einem der Ansprüche 9 bis 16, dadurch gekennzeichnet, daß die biologisch verträgliche anionische polymere Substanz einen polysacchariden Gummi mit freien Säuregruppen enthält.

18. Verfahren nach Anspruch 17, dadurch gekennzeichnet, daß der polysaccharide Gummi ein Natriumalginat ist.

19. Verfahren nach einem der Ansprüche 9 bis 16, dadurch gekennzeichnet, daß die biologisch verträgliche anionische polymere Substanz ein Polyvinylalkohol ist, der freie Hydroxylgruppen oder eine Polymilchsäure mit freien Säuregruppen aufweist.

20. Verfahren nach einem der Ansprüche 9 bis 18, dadurch gekennzeichnet, daß die reversibel gelierbare wasserlösliche Substanz Natriumalginat, das kationische Polymer Polylysin und das anionische Polymer Natriumalginat enthält.

21. Verfahren nach einem der Ansprüche 9 bis 18 und 20, dadurch gekennzeichnet, daß die reversibel gelierbare wasserlösliche Substanz Natriumalginat ist und die Viskosität der wässrigen Lösung des Natriumalginats, welche im Verfahrensschritt (a) verwendet wird, wenigstens dafür ausreicht, die Bildung von im wesentlichen sphärischen vorläufigen Kapseln zu bewirken.

22. Verfahren nach Anspruch 21, dadurch gekennzeichnet, daß die wässrige natriumalginatlösung eine Viskosität von wenigstens 30 mPa.s aufweist.

23. Verfahren nach einem der Ansprüche 9 bis 22, gekennzeichnet durch einen weiteren Verfahrensschritt, daß das innerhalb der semipermeablen Membran sich befindende Gel nach Verfahrensschritt (e) wieder verflüssigt wird.

**Revendications**

1. Une microcapsule biocompatible convenant pour l'implantation dans un corps animal et ayant un diamètre de 50 à 2000 micromètres, cette microcapsule comprenant un noyau macromoléculaire sphérique contenant un tissu vivant ou des cellules individuelles de celui-ci, ledit noyau étant entouré par un membrane semi-perméable biocompatible consistant en couches s'interpénétrant d'un polyamino-acide biocompatible et d'une substance polymère hydrosoluble gélifiable de façon réversible et ayant des groups acides libres, le polyamino-acide et la substance polymère ayant réagi l'un avec l'autre ioniquement, et ladite membrane étant perméable aux substances nutritives et à l'oxygène et permettant à ceux-ci de s'écouler d'un corps dans lequel la microcapsule est implantée vers ledit tissu vivant ou les cellules individuelles de celui-ci et permettant aux produits métaboliques dudit tissu ou desdites cellules de s'écouler de ceux-ci vers ledit corps et étant imperméable audit tissu ou auxdites cellules, caractérisée en ce que la membrane a une épaisseur de 5 à 20 micromètres, est sous forme d'un hydrogel ayant une teneur totale en eau à l'intérieur de la structure de la membrane d'au moins 20% en poids, a une surface externe biocompatible chargée négativement, formée par réaction ionique de groupes amino libres dudit polyamino-acide avec un matière polymère ayant des groupes libres chargés négativement, et résiste à la dégradation et demeure perméable in vivo pendant au moins deux mois.

2. Une microcapsule telle que revendiquée dans la revendication 1, dans laquelle la matière du noyau est choisie parmi les îlots de Langerhans, le tissu hépatique ou des cellules individuelles de ceux-ci.

3. Une microcapsule telle que revendiquée dans la revendication 2, dans laquelle le tissu vivant est les îlots de Langerhans ou une fraction de ces îlots, ce qui fait que de l'insuline s'écoule de la microcapsule, et ladite membrane semi-perméable demeure efficace pendant au moins trois mois lorsqu'on implante la microcapsule pour régler les niveaux de sucre dans le sang, dans le corps dans lequel la microcapsule est implantée.

4. Une microcapsule telle que revendiquée dans l'une quelconque des revendications précédentes, dans laquelle ladite matière polymère hydrosoluble gélifiable de façon réversible est un polysaccharide.

5. Une microcapsule telle que revendiquée dans la revendication 4, dans laquelle ledit polysaccharide est un alginate.

6. Une microcapsule telle que revendiquée dans l'une quelconque des revendications précédentes, dans laquelle ledit polyamino-acide est la polylysine.

7. Une microcapsule telle que revendiquée dans l'une quelconque des revendications précédentes, dans laquelle ladite matière polymère chargée négativement est un polysaccharide, l'alcool polyvinylique ou l'acide polylactique.

8. Une microcapsule telle que revendiquée dans la revendication 7, dans laquelle ledit polysaccharide est un alginate.

9. Un procédé d'encapsulation d'une matière de noyau avec une membrane semi-perméable pour former des microcapsules ayant un diamètre de 50 à 2000 micromètres, ce procédé comprenant les étapes suivantes: (a) on place la matière dans une solution aqueuse d'une substance polymère hydrosoluble qui peut être gélifiée de façon réversible et qui a des groupes acides libres; (b) on met la solution sous forme de gouttelettes; (c) on gélifie les gouttelettes pour produire des capsules temporaires séparées les unes des autres et conservant leur forme; (d) on forme des membranes semi-perméables autour des capsules temporaires par mise en contact de celles-ci avec un polymère cationique contenant des groupes amino libres pour provoquer une réaction ionique avec les groupes acides dans une couche de surface de la capsule, et (e) on met en contact les microcapsules formées dans l'étape (d) avec une matière polymère anionique biocompatible qui contient des groupes libres chargés négativement capables de réagir ioniquement avec les groupes amino libres dans une couche de surface de la microcapsule, caractérisé en ce que ledit polymère anionique forme sur les microcapsules un revêtement externe ayant une surface externe chargée négativement et en ce que la membrane est un hydrogel ayant une teneur totale en eau à l'intérieur de la structure de la membrane d'au moins 20% en poids et en ce que le contact avec le polymère cationique est suffisant pour former une membrane qui a une épaisseur de 5 à 20 micromètres et qui résiste à la dégradation et qui demeure perméable in vivo pendant au moins deux mois.

10. Procédé tel que revendiqué dans la revendication 9, dans lequel la matière de noyau comprend un tissu vivant ou des cellules individuelles de celui-ci sous forme d'une suspension finement divisée dans ladite solution aqueuse de l'étape (a).

11. Un procédé tel que revendiqué dans la revendication 10, dans lequel ledit tissu vivant comprend des îlots de Langerhans, ce qui fait que les microcapsules produites par le procédé peuvent être utilisées pour régler les teneurs en sucre du sang dans des corps animaux diabétiques dans lesquels elles sont implantées.

12. Un procédé tel que revendiqué dans l'une quelconque des revendications 9 à 11, dans lequel ladite substance polymère hydrosoluble gélifiable de façon réversible est une gomme de polysaccharide.

13. Un procédé tel que revendiqué dans la revendication 12, dans lequel ladite gomme est un alginate de métal alcalin.

14. Un procédé tel que revendiqué dans l'une quelconque des revendications 9 à 13, dans lequel ledit polymère cationique a un poids moléculaire de 11 000 à 400 000 daltons.

15. Un procédé tel que revendiqué dans la revendication 14, dans lequel ledit polymère cationique a un poids moléculaire de 11 000 à 100 000 daltons.

16. Un procédé tel que revendiqué dans l'une quelconque des revendications 9 à 15, dans lequel ledit polymère cationique est la polylysine.

17. Un procédé tel que revendiqué dans l'une quelconque des revendications 9 à 16, dans lequel ladite matière polymère anionique biocompatible comprend une gomme de polysaccharide contenant des groupes acides libres.

18. Un procédé tel que revendiqué dans la revendication 17, dans lequel ladite gomme de polysaccharide est l'alginate de sodium.

19. Un procédé tel que revendiqué dans l'une quelconque des revendications 9 à 16, dans lequel ladite matière polymère anionique biocompatible est un alcool polyvinylique ayant des groupes hydroxyle libres ou un acide polylactique contenant des groupes acides libres.

20. Un procédé tel que revendiqué dans l'une quelconque des revendications 9 à 18, dans lequel ladite substance hydrosoluble gélifiable de façon réversible comprend l'alginate de sodium, ledit polymère cationique comprend la polylysine et ledit polymère anionique comprend l'alginate de sodium.

21. Un procédé tel que revendiqué dans l'une quelconque des revendications 9 à 18 et 20, dans lequel ladite substance hydrosoluble gélifiable de façon réversible est l'alginate de sodium et la viscosité de la solution aqueuse d'alginate de sodium utilisée dans l'étape (a) est au moins suffisante pour provoquer la formation de capsules temporaires sensiblement sphériques.

22. Un procédé tel que revendiqué dans la revendication 21, dans lequel ladite solution aqueuse d'alginate de sodium a une viscosité d'au moins 30 mPa.s.

23. Un procédé tel que revendiqué dans l'une quelconque des revendications 9 à 22, incluant l'étape supplémentaire consistant à reliquéfier le gel à l'intérieur de la membrane semi-perméable à la suite de l'étape (e).